Europäisches Patentamt

European Patent Office

Office européen des brevets

(11) Publication number: **0 556 905 A1**

## EUROPEAN PATENT APPLICATION

(21) Application number: **93200386.6**

(22) Date of filing: **11.02.93**

(51) Int. Cl.5: **C12N 1/18**, A21D 8/04, C12N 1/20, //(C12N1/18, C12R1:865)

(30) Priority: **17.02.92 EP 92200445**

(43) Date of publication of application: **25.08.93 Bulletin 93/34**

(84) Designated Contracting States: **BE CH DE FR GB LI NL**

(71) Applicant: **GIST-BROCADES N.V. Wateringseweg 1 P.O. Box 1 NL-2600 MA Delft(NL)**

(72) Inventor: **Osinga, Klaas Anne Van der Rijnplantsoen 3 NL-2253 TC Voorschoten(NL)**

(74) Representative: **Matulewicz, Emil Rudolf Antonius, Dr. et al Gist-Brocades NV Patents and Trademarks Department Wateringseweg 1 P.O. Box 1 NL-2600 MA Delft (NL)**

(54) **Cold-sensitive strain.**

(57) A microorganism is disclosed having a a cold-sensitive step that blocks a microbial activity in a metabolic pathway at a non-permissive temperature. Further the use of the microorganism as well as a process for obtaining said microorganism is disclosed.

Rank Xerox (UK) Business Services
(3.10/3.6/3.3.1)

The present invention relates to a cold-sensitive strain, which is not able to have a specified microbial activity at a non-permissive temperature.

The procedure of producing refrigerated dough products with chemical leavening involves mixing of a dough, different line operations and putting the formed pieces of dough into a can.

During a proofing step the product expands and expels the air from the can. Dough expansion finally results in a plugging of the can and the formation of an overpressure.

Simple replacement of chemical leavening by common bakers' yeast is not applicable as the total volume of gas produced is sufficient to burst the can, even at lower temperature ($0\text{-}10\,^\circ$C).

Calculations based on the average composition of flour indicate that a total volume of about 1200 ml of gas can be produced from 100 g of dough by fermentation.

These calculations indicate that in order to replace chemical leavening by yeast the volume of gas produced in the can during refrigerated storage has to be reduced substantially.

To inactivate microorganisms in e.g. food several inactivation methods are known such as high temperature treatment (pasteurization or sterilisation) and gamma irradiation of the product. At first glance, these methods seem attractive but all show drawbacks in the industrial application. High temperature inactivation will commonly necessitate a process adaptation and in case of food products such as dough high temperatures negatively affect the dough structure.

Gamma irradiation of the final product is not a fully accepted method of food preservation. All the inactivation processes have in common that they irreversibly destroy the microbiological activity of the present cells.

According to the present invention a method is disclosed to inactivate a strain by way of low temperature. When lowering the temperature to e.g. $0\text{-}10\,^\circ$C, many microorganisms will be in a non-growing or slow-growing stage. However some metabolic activity, although reduced, is still present. For example, bakers' yeast converts in dough under non-growing conditions still some sugar into $CO_2$ even at a storage temperature of $4\,^\circ$C.

We have found now that introducing a cold-sensitive step in the metabolic pathway in question of a microorganism blocks the corresponding microbial activity at a non-permissive temperature. Preferably the trigger shows reversible working, viz. when after an inactivation period at a non-permissive temperature, the microorganism is brought to normal conditions wherein the original activity of the microorganism is substantially restored and preferably is almost of the same level of before the inactivation period. The use of a cold-sensitive bakers' yeast in refrigerated dough products is attractive since the trigger of inactivation is part of the normal process viz. storage of dough at low temperature.

In case of a bakers' yeast the cold-sensitive step can be introduced in the glycolysis pathway. This will block the fermentation of sugars at the non-permissive temperature and hence the production of gas. Another option to introduce the trigger is to interfere in the cell structure. Actine and tubuline are present as fibre-like structures in the cell. By introducing a cold-sensitive trigger in these structures the cell structure can be altered at the non-permissive temperature. For example, at these lowered temperature the fibre-like structure will lose their strength and the cell will collapse. Preferably the trigger is chosen in such a way that this collapse is reversible.

Several techniques are nowadays available to construct a strain with the desired mutation such as rDNA techniques or gene disruption. In case of bakers' yeast a cold-sensitive strain can be obtained by interfering in the glycolysis pathway. Preferably, the starting strain is haploid (a haploid strain is more convenient to isolate/analyse such recessive mutants) and a good bakers' yeast strain. These yeast mutants are then tested with respect to gas production during storage conditions. The most desired mutants do not produce any gas anymore at low temperature.

Cold-sensitive bakers' yeast mutants in the glycolysis using mutagenesis can for example be obtained by:

(a) Mutagenesis of yeast cells and selection of cold-sensitive ($C^s$) mutants in glycolysis according to the following tests: the permissive temperature chosen is $28\,^\circ$C, the non-permissive temperature chosen is $10\text{-}14\,^\circ$C, at permissive temperature the yeast will grow on glucose and on glycerol and ethanol; at non-permissive temperature: no growth on glucose but the yeast will grow on glycerol and ethanol.

(b) Mutagenesis of yeast cells and selection of a temperature sensitive ($T^s$) mutant in glycolysis (for example no growth at $35\,^\circ$C). The next step is to screen for intragenic pseudorevertants of this mutant, able to grow again at $35\,^\circ$C. It is anticipated that some of these intragenic pseudorevertants are cold-sensitive mutants as well.

(c) In vitro mutagenesis on a cloned glycolytic gene. After transformation into yeast (for instance via gene-disruption), selection as described above. Depending on the overall performance (production, baking) of a resulting haploid strain with a cold-sensitive glycolysis, we can construct a diploid with a

2

cold-sensitive glycolysis or transfer the cold-sensitive mutation to an industrial strain.

It will be appreciated that for other microorganisms in order to block their microbial activity similar mutations can be made, introducing the trigger in a suitable metabolic pathway.

## Legend to the Figure

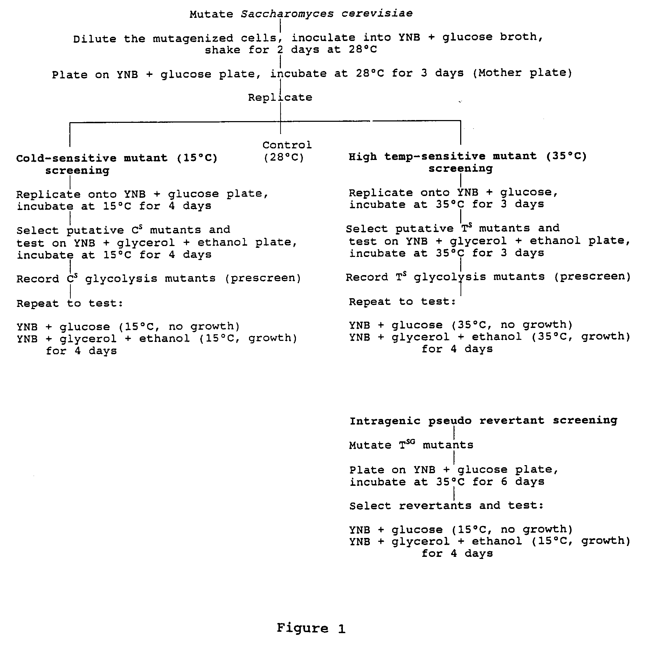

Figure 1: outline of a possible screening procedure.

## Example 1

## Mutagenesis and screening for glycolytic mutants

The mutagenesis and screening protocol is outlined in Figure 1. In essence, two routes are being followed simultaneously. Firstly, a direct screen for $C^s$ mutants. Secondly, via the isolation of intragenic pseudorevertants of glycolytic $T^s$ mutants. It is anticipated that some of these revertants are $C^s$ mutants as well.

We ran four mutation cycles (Experiment 1-4) for screening conditional yeast mutants using NTG and UV. About 132,000 mutated colonies of Saccharomyces cerevisiae (X2180-1B YGSC (Yeast Genetic Stock Center), University of California, Berkley CA 94720, USA) were examined for both 15°C $C^s$ and 35°C $T^s$ glycolysis mutants. In these experiments YNB was used as base medium and Bacto-agar for solidification. The results of the four screening cycles are given in Tables 1-4, which list the mutation data, number of the selected mutants and the confirmed characteristics of these mutants.

We obtained the following categories of the obtained mutants:

- $C^{SG}$     Strains 1-1, 1-3, 3-1, 3-2, 3-4, 3-5, 3-6. Strain 1-3 is $C^{SG}$, although it was screened from $T^s$ selection. In the confirmation test, it showed no $T^s$ characteristic, but on the contrary the $C^{SG}$ characteristic.

- $(C^{SG})$     Leaky* $C^{SG}$ mutants. Strains 4-1 and 1-4 (the latter is obtained from $T^s$ selection).

- $T^{SG}$     Strains 1-5, 1-6, 2-2, 4-2, 4-4, 4-5, 4-8. Note that strains 1-5, 4-2 and 4-4 are also affected with respect to $C^s$ (leaky $C^{SG}$);

- $(T^{SG})$     Leaky $T^{SG}$ mutants. Strains 4-6 and 4-7.

* The leaky mutant had trace growth on YNB and glucose plate at the restricted temperature.

3

TABLE 1: DATA OF EXPERIMENT 1

| Mutagen & killing rate (%) | | NTG 95% |
|---|---|---|
| Result | $C^{SG}$ mutant/screened mutant | 1/18902 |
| | $T^{SG}$ mutant/screened mutant | 2/18902 |
| | $C^{SG}$ mutant from $T^{SG}$ screen | 2 |
| | revertant number | proceeding |

Confirmed characteristics of the selected mutants

| Strain | | Media | | | | | | Confirmation of characteristic |
|---|---|---|---|---|---|---|---|---|
| | | YNB + glucose | | | YNB + glycerol + ethanol | | | |
| No. | pre-screen | 15°C | 28°C | 35°C | 15°C | 28°C | 35°C | |
| 1-1 | $C^S$ | x | o | o | o | o | o | $C^{SG}$ |
| 1-3 | $T^S$ | x | o | o | o | o | o | $C^{SG}$ |
| 1-4 | $T^S$ | △ | o | o | o | o | o | $(C^{SG})$ |
| 1-5 | $T^S$ | △ | o | x | o | o | o | $T^{SG}$ $(C^{SG})$ |
| 1-6 | $T^S$ | o | o | x | o | o | o | $T^{SG}$ |
| wild type | | o | o | o | o | o | o | |

Remark:
o   normal growth     $C^{SG}$    15°C sensitive glycolysis mutant
△   trace growth      $(C^{SG})$   15°C leaky sensitive glycolysis mutant
x   no growth        $T^{SG}$    35°C sensitive glycolysis mutant
                     $(T^{SG})$   35°C leaky sensitive glycolysis mutant


TABLE 2: DATA OF EXPERIMENT 2

| Mutagen & killing rate (%) | | NTG 99.5% |
|---|---|---|
| Result | $C^{SG}$ mutant/screened mutant | 0/53161 |
| | $T^{SG}$ mutant/screened mutant | 1/53161 |
| | $C^{SG}$ mutant from $T^{SG}$ screen | 0 |
| | revertant number | proceeding |

Confirmed characteristics of the selected mutants

| Strain | | Media | | | | | | Comfirmation of characteristic |
|---|---|---|---|---|---|---|---|---|
| | | YNB + glucose | | | YNB + glycerol + ethanol | | | |
| No. | pre-screen | 15°C | 28°C | 35°C | 15°C | 28°C | 35°C | |
| 2-2 | $T^S$ | o | o | x | o | o | o | $T^{SG}$ |
| wild type | | o | o | o | o | o | o | |

Remark:
o   normal growth     $C^{SG}$    15°C sensitive glycolysis mutant
△   trace growth      $(C^{SG})$   15°C leaky sensitive glycolysis mutant
x   no growth        $T^{SG}$    35°C sensitive glycolysis mutant
                     $(T^{SG})$   35°C leaky sensitive glycolysis mutant

TABLE 3: DATA OF EXPERIMENT 3

| Mutagen & killing rate (%) | | NTG 88.6% |
|---|---|---|
| Result | $C^{SG}$ mutant/screened mutant | 5/45352 |
| | $T^{SG}$ mutant/screened mutant | 0/45352 |
| | $C^{SG}$ mutant from $T^{SG}$ screen | 0 |
| | revertant number | proceeding |

Confirmed characteristics of the selected mutants

| Strain | | Media | | | | | | Confirmation of characteristic |
|---|---|---|---|---|---|---|---|---|
| | | YNB + glucose | | | YNB + glycerol + ethanol | | | |
| No. | pre-screen | 15°C | 28°C | 35°C | 15°C | 28°C | 35°C | |
| 3-1 | $C^S$ | x | o | | o | | | $C^{SG}$ |
| 3-2 | $C^S$ | x | o | | o | | | $C^{SG}$ |
| 3-4 | $C^S$ | x | o | | o | | | $C^{SG}$ |
| 3-5 | $C^S$ | x | o | | o | | | $C^{SG}$ |
| 3-6 | $C^S$ | x | o | | o | | | $C^{SG}$ |
| wild type | | o | o | | o | | | |

Remark:
o   normal growth
△   trace growth
x   no growth

$C^{SG}$      15°C sensitive glycolysis mutant
$(C^{SG})$   15°C leaky sensitive glycolysis mutant
$T^{SG}$      35°C sensitive glycolysis mutant
$(T^{SG})$   35°C leaky sensitive glycolysis mutant

TABLE 4: DATA OF EXPERIMENT 4

| Mutagen & killing rate (%) | | NTG 99.6%<br>UV 99.999% (separatedly) | |
|---|---|---|---|
| Result | $C^{SG}$ mutant/screened mutant | 2/7350 (NTG); 0/7854 (UV) | |
| | $T^{SG}$ mutant/screened mutant | 5/7350 (NTG); 0/7850 (UV) | |
| | $C^{SG}$ mutant from $T^{SG}$ screen | 1 | |
| | revertant number | proceeding | |

Confirmed characteristics of the selected mutants

| Strain | | Media | | | | | | Confirmation of characteristic |
|---|---|---|---|---|---|---|---|---|
| | | YNB + glucose | | | YNB + glycerol + ethanol | | | |
| No. | pre-screen | 15°C | 28°C | 35°C | 15°C | 28°C | 35°C | |
| 4-1 | $C^S$ | △ | o | o | o | | o | $(C^{SG})$ |
| 4-2 | $C^S$ | △ | o | x | o | | o | $T^{SG}$ $(C^{SG})$ |
| 4-4 | $T^S$ | △ | o | x | o | | o | $T^{SG}$ $(C^{SG})$ |
| 4-5 | $T^S$ | o | o | x | o | | o | $T^{SG}$ |
| 4-6 | $T^S$ | o | o | △ | △ | | o | $(T^{SG})$ |
| 4-7 | $T^S$ | o | o | △ | o | | o | $(T^{SG})$ |
| 4-8 | $T^S$ | o | o | x | o | | o | $T^{SG}$ |
| wild type | | o | o | o | o | | o | |

Remark:
o    normal growth         $C^{SG}$      15°C sensitive glycolysis mutant
△    trace growth          $(C^{SG})$    15°C leaky sensitive glycolysis mutant
x    no growth             $T^{SG}$      35°C sensitive glycolysis mutant
                           $(T^{SG})$    35°C leaky sensitive glycolysis mutant

## Claims

1. A microorganism, in isolated form, having a cold-sensitive step that blocks a microbial activity in a metabolic pathway at a non-permissive temperature.

2. A microorganism according to claim 1 which is a bakers' yeast.

3. A microorganism according to claim 1, having a cold-sensitive step at a non-permissive temperature of 0 - 10°C.

4. A composition of matter which comprises a microorganism according to claim 1 which is kept at a non-permissive temperature.

5. A composition of matter of claim 4 which is a dough.

6. Use of a microorganism according to claim 1 or a composition according to claim 4 in an industrial process.

7. Process for obtaining a microorganism according to claim 1 which comprises the use of mutation techniques.

8. Process according to claim 7 which comprises the use of rDNA techniques and gene disruption.

```
                    Mutate Saccharomyces cerevisiae
                                   |
        Dilute the mutagenized cells, inoculate into YNB + glucose broth,
                          shake for 2 days at 28°C
                                   |
        Plate on YNB + glucose plate, incubate at 28°C for 3 days (Mother plate)
                                   |
                              Replicate
                                   |
        _____|_____
       |                           |                           |
                                Control
  Cold-sensitive mutant (15°C)   (28°C)      High temp-sensitive mutant (35°C)
       screening                                        screening
          |                                                |
  Replicate onto YNB + glucose plate,          Replicate onto YNB + glucose,
  incubate at 15°C for 4 days                  incubate at 35°C for 3 days
          |                                                |
  Select putative Cˢ mutants and               Select putative Tˢ mutants and
  test on YNB + glycerol + ethanol plate,      test on YNB + glycerol + ethanol plate,
  incubate at 15°C for 4 days                  incubate at 35°C for 3 days
          |                                                |
  Record Cˢ glycolysis mutants (prescreen)     Record Tˢ glycolysis mutants (prescreen)
          |                                                |
  Repeat to test:                              Repeat to test:

  YNB + glucose (15°C, no growth)              YNB + glucose (35°C, no growth)
  YNB + glycerol + ethanol (15°C, growth)      YNB + glycerol + ethanol (35°C, growth)
      for 4 days                                   for 4 days
```

**Intragenic pseudo revertant screening**

Mutate $T^{SG}$ mutants

Plate on YNB + glucose plate,
incubate at 35°C for 6 days

Select revertants and test:

YNB + glucose (15°C, no growth)
YNB + glycerol + ethanol (15°C, growth)
        for 4 days

Figure 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 487 878 (SOCIETE DES PRODUITS NESTLE S.A.)<br>* the whole document * | 1-8 | C12N1/18<br>A21D8/04<br>C12N1/20<br>//(C12N1/18,<br>C12R1:865) |
| P,X | WO-A-9 301 724 (THE PILLSBURY COMPANY)<br>* the whole document * | 1-8 | |
| A | EP-A-0 229 976 (UNIVERSAL FOODS CORPORATION)<br>* page 4, line 6 - page 5, line 5 * | 1-2,7-8 | |
| A | US-A-4 547 374 (Y. NAKATOMI ET AL.) | | |
| A | WO-A-8 703 006 (GENETICS INSTITUTE INC.) | | |
| A | CEREAL CHEMISTRY<br>vol. 64, no. 4, August 1987, MINNEAPOLIS US<br>pages 269 - 275<br>A. HINO ET AL. 'New freeze-tolerant yeast for frozen dough preparations.' | | |
| A | PATENT ABSTRACTS OF JAPAN<br>vol. 14, no. 304 (C-734)(4247) 29 June 1990<br>& JP-A-21 00 673 ( TAIJI OSHIMA ) 12 April 1990<br>* abstract * | | |

TECHNICAL FIELDS SEARCHED (Int. Cl.5)

C12N
A21D
C12R

The present search report has been drawn up for all claims

| Place of search | Date of completion of the search | Examiner |
|---|---|---|
| THE HAGUE | 03 JUNE 1993 | BEVAN S.R. |

EPO FORM 1503 03.82 (P0401)